# FASCICULE DE BREVET EUROPEEN

(11) **EP 1 543 846 B1**
(45) Date de publication et mention de la délivrance du brevet: **19.08.2009**
(21) Numéro de dépôt: 03029118.1
(22) Date de dépôt: 18.12.2003
(51) Int. Cl.: A61L 15/32, A61L 24/10

(54) **Hydrogel biorésorbable réticulé à base d'albumine**
Bioresorbierbares vernetztes Hydrogel bestehend aus Albumin
Albumin-based bioresorbable cross-linked hydrogel

(43) Date de publication de la demande: 22.06.2005
(73) Titulaire: Turzi, Antoine, 1146 Mollens (CH)
(72) Inventeur: Turzi, Antoine, 1146 Mollens (CH); Fortier, Guy, Montréal, Québec H4A2Z3 (CA); Liu, Yaode, Montréal, Québec H4A2Z3 (CA)
(74) Mandataire: reuteler & cie SA

(56) Documents cités:
- WO-A-00/10618
- WO-A-95/15352
- WO-A-99/66964
- FR-A- 2 796 558
- US-A- 5 583 114
- US-A- 5 791 352
- GAYET J-C ET AL: "High water content BSA-PEG hydrogel for controlled release device: Evaluation of the drug release properties" JOURNAL OF CONTROLLED RELEASE, ELSEVIER SCIENCE PUBLISHERS B.V. AMSTERDAM, NL, vol. 38, no. 2, 1 février 1996 (1996-02-01), pages 177-184, XP004037403 ISSN: 0168-3659

## Description

La présente invention concerne un gel hydrophile, dissociable en milieu aqueux, un procédé pour la préparation de cet hydrogel, ainsi qu'une membrane de séparation biorésorbable utilisable notamment en chirurgie et plus particulièrement dans le but de minimiser et/ou d'empêcher la formation d'adhérences post-chirurgicales.

La formation d'adhérences post-opératoire est très fréquente lors de chirurgies cardiaques, abdominales ou pelviennes. Ces adhérences peuvent dégénérer en pathologies obstructives en fonction de leur étendu et de leur sévérité. Lors de chirurgies abdominales, la formation d'adhésions peut par exemple compromettre la mobilité des organes et induire des douleurs chroniques chez le patient. Les adhésions sont responsables de 49 à 74%,des obstructions du petit intestin, de 10 à 15% des cas d'infertilité et de 20 à 50% des douleurs pelviennes chroniques chez la femme. De plus, lors d'une ré-opération, la présence d'adhésions peut augmenter de manière significative la complexité et la durée de la chirurgie, de même que les complications post-opératoires. Les mêmes conclusions s'appliquent aussi pour les chirurgies cardiaques, pulmonaires ou thoraciques. La présence d'adhésions est un facteur influant aussi bien sur le risque opératoire (mortalité et morbidité) que sur les coûts du traitement.

La formation d'adhérences apparaît durant le processus de réparation naturelle des tissus sains et sont constitués par des attachements fibreux entre les organes visés par la chirurgie ou encore entre une membrane séparatrice comme le péritoine ou le péricarde et les organes sous-jacents. A titre d'exemple, lorsque le péritoine est endommagé durant une chirurgie, de la fibrine s'accumule à la surface de ce dernier. Ces bandes de fibrine agissent comme une colle et induisent une adhésion entre l'organe et la membrane. Dans les conditions idéales, suivant la réparation du site, la fibrine présente au site des dommages mésothéliales serait détruit par la plasmine. La plasmine résulte de l'action de l'activateur tissulaire du plasminogène (cellules mésothéliales) sur le plasminogène sanguin. La fibrinolyse ainsi que la cascade de coagulation sont mises à contribution dans cette pathologie. Un recrutement de fibroblastes et leur prolifération ainsi que la présence de cellules inflammatoires sont aussi observées, parallèlement à la déposition de collagène. Un mécanisme angiogénèse sous contrôle des cellules endothéliales serait éventuellement impliqué dans la formation des adhésions.

Une des stratégies innovantes actuellement proposée pour limiter la formation d'adhésions, sans pour autant interférer avec le mécanisme naturel de réparation, consiste à insérer entre la membrane endommagée et les organes sous-jacents, un feuillet, séché ou non, de polymère synthétique ou naturel, réticulé chimiquement ou non. Le feuillet sec au contact des tissus s'hydrate et subit une érosion par un mécanisme lent de dissolution ou par d'hydrolyse. Ce processus conduit à la résorption complète du feuillet dans les semaines suivant son implantation. La formation des adhérences qui débute dès la fin de l'opération prend fin environ 7 jours plus tard. Ainsi donc, si au cours de la première semaine une barrière physique est placée par exemple entre le péricarde et l'épicarde, ou bien encore entre le péritoine et les organes du système gastro-intestinal, ou même entre la plèvre et le poumon, le nombre de sites d'adhésion devrait y être minimisé. A la disparition de la barrière, des adhésions rattachées par une seule de leurs extrémités devraient être observées. Cette barrière ou séparateur doit posséder des qualités de biodégradabilité, de malléabilité, de biocompatibilité, être non abrasive et facile à synthétiser.

Le terme hydrogel réfère par définition à un matériau polymérique naturel ou artificiel qui a la propriété de retenir plusieurs fois son poids en eau, ce qui lui confère une ressemblance en terme d'hydratation avec les tissus vivants. Ainsi, il apparaît que les hydrogels peuvent jouer le rôle de barrière, i.e. de feuillets séparateurs.

La majorité des films polymériques hydratés sont obtenus par réticulation chimique de polymères hydrophiles naturels ou synthétiques en présence d'agents bifonctionnels ou par polymérisation de monomères, ce qui leur confèrent une stabilité structurale élevée et conséquemment une faible biodégradabilité. De tels films sont utiles en usage externe comme pansement cutané et pour la libération contrôlée de médicament. In vivo, leurs utilisations et applications sont limitées par le fait qu'ils se dégradent très lentement, lorsqu'il le font. Il existe donc un besoin dans le domaine médical pour des films d'hydrogel hydratés biodégradables qui pourraient être utilisés in vivo lors de chirurgies afin de diminuer les adhérences et même pour permettre la libération contrôlée de médicaments in situ. Plusieurs types de films polymériques secs existent à des fins de libération contrôlée de médicaments et qui se désagrègent par dissolution. Ces films secs ne peuvent être implantés dans certaines parties du corps humain, car ils sont solides, non flexibles et abrasifs et requièrent un lieu d'implantation sans contrainte mécanique, au contraire d'un hydrogel hydraté qui de part sa nature est flexible et non abrasif. Le film d'hydrogel hydraté et biodégradable peut être positionné dans toutes les parties du corps, même celles soumises à un stress mécanique tels les articulations, le coeur et la cavité péritonéale. Suite à sa dégradation, plus aucune trace de l'hydrogel ne devrait être retrouvée au site d'implantation, éliminant tout risque relié à la bio accumulation du polymère.

La majorité des films polymériques hydratés sont obtenus par réticulation chimique de polymères hydrophiles naturels ou synthétiques en présence d'agents bifonctionnels ou par polymérisation de monomères ce qui leur confèrent une stabilité structurale élevée et conséquemment une faible biodégradabilité. Ainsi, pour des utilisations in vivo, les films d'hydrogel doivent se dégrader dans un temps suffisamment court, c'est à dire dans un temps inférieur au temps requis pour la formation d'une capsule fibreuse qui résulte de la réaction inflammatoire cellulaire de l'hôte sur l'implant. On parle ici d'un temps de dégradation d'une vingtaine de jours à quelques semaines.

Des membranes amniotiques humaines irradiées (Young et al., Fertil. Steril., 55:624-628,1991) ont été utilisées avec un certain succès chez le lapin modèle lors de chirurgies pelviennes. Cependant des problèmes éthiques et d'approvisionnement ont limité son développement. Deux hydrogels ont été évalués chez le rat modèle (West et Hubbell, Biomaterials, 16 : 1153-1156, 1995), le premier photo-polymérisé in situ et fait de poly(éthylène glycol)-co-acide lactique diacrylique et le deuxième réticulé physiquement et fait de poly(éthylène glycol)-co-poly(propylène glycol) et Poloxamer 407. Une diminution des adhérences de 75 et de 38% a été observée, respectivement; les temps de biodégradation respectifs étaient de 4 jours pour le premier et de 2 jours pour le second. Plus récemment, une membrane d'hydrogel obtenue par pulvérisation de deux liquides contenant du poly(éthylène glycol) sur le site chirurgical pelvien chez le porc a permis une réduction significative de 60% du nombre et de l'intensité des adhésions. Le revêtement d'hydrogel s'est résorbé en 5 jours (Ferland et al, Hum. Reprod., 16 : 2718.2723, 2001 ).

Des hydrogels d'acide hyaluronique réticulé ont donné une réduction significative des adhésions et des ré-adhésions abdominales chez le lapin, cependant la dégradabilité du polymère n'a pas été discutée (Osada et al., J. Int. Med. Res., 27:233-241, 1999). Lors d'une étude clinique, des feuillets d'acide hyaluronique et de carboxyméthylcellulose ont permis une diminution de 51 % de l'incidence des adhérences et de 87% de la sévérité de ces adhérences (Becker et al., J. Am. Coll. Surg., 183 : 297-306, 1996) après une chirurgie abdominale.

Pour les chirurgies cardiaques, les premières membranes pour limiter la formation d'adhésions épicarde-péricarde ont été faites de poly(2-hydroxyéthyle méthacrylate) renforcées avec du poly(éthylène térephthalate) (Walker et al., Asaio J. 38:M550-554, 1992). Ces membranes d'hydrogel assez rigides n'étaient pas biodégradables et étaient sujettes à la calcification après 9 mois d'implantation chez le chien. Les membranes de poly(hydroxybutyrate) ont donné de bons résultats chez le mouton, cependant elles étaient résorbées que très lentement par les macrophages après plus de 30 mois (Malm et al., J. Thorac. Cardiovasc. Surg. 104:600-607, 1992). Des expériences similaires chez le chien, utilisant des membranes résorbables à base de poly(éthylène glycol) et d'acide poly(lactique) ont montré un abaissement des adhérences, cependant leur biodégradabilité était très rapide et la résorption était complète après quelques jours (Okuyama et al., Ann. Thorac. Surg. 68:913-918, 1999). Des membranes résorbables de collagène élastine (Fierez et al., J. Thorac. Cardiovasc. Surg., 117:185,1999) ont été utilisées avec succès pour limiter le nombre d'adhésions lors de chirurgie cardiaque chez l'humain. Plus récemment, des feuillets de N-O carboxyméthyl-chitosan ont été utilisés aux même fins, mais chez le lapin, et ils ont permis une réduction très significative du nombre d'adhésions, cependant on n'y discute pas de la biodégradabilité de ces feuillets (Krause et al., J. Invest. Surg., 14:93-97, 2001).

Le but de la présente invention consiste à fournir un nouveau type d'hydrogel destiné à former une membrane de séparation biodégradable, visant à remédier aux inconvénients des films polymériques connus, et qui présente une excellente biocompatibilité, une bonne dégradabilité in vivo, des caractéristiques viscoélastiques qui facilitent sa manipulation et une absence d'effet abrasif.

Un premier objet de cette invention visant à atteindre le but précité est donc un hydrogel physiquement réticulé, hydrophile, gonflable dans l'eau et lentement dissociable en milieu aqueux, qui consiste en protéines sanguines d'albumine associées sous forme de gel en milieu basique.

Un second objet de l'invention consiste en une membrane de séparation biorésorbable qui est formée d'un film de l'hydrogel susmentionné.

Un troisième objet de l'invention est un procédé pour la préparation de l'hydrogel susmentionné, qui consiste à mélanger une solution aqueuse d'albumine protéique avec une base, puis à laisser reposer le mélange jusqu'à la polymérisation des macromolécules d'albumine.

Enfin, l'invention a également pour objet l'utilisation de la membrane de séparation en chirurgie vétérinaire et humaine, ainsi que pour l'administration contrôlée d'une substance thérapeutiquement active.

Les protéines sanguines d'albumine peuvent être isolées du plasma ou des sérums d'origine animale (boeuf, lapin, porc, etc.) ou humaine.

L'albumine d'origine humaine peut être obtenue d'une banque de sang ou par génie génétique, ou bien être de nature autologue. Dans ce dernier cas, il apparaît que l'acceptabilité par le patient sera nettement meilleure envers l'implant, de par la composition autologue de la membrane.

En ce qui concerne le procédé de préparation de l'hydrogel selon l'invention, il est de préférence caractérisé en ce que la solution aqueuse d'albumine est comprise entre 10 et 20% (poids/vol.), que la base est du NaOH 5N et que le rapport entre le NaOH et la solution d'albumine est de 0.5 à 2.0% (poid s/vol.), et par le fait que la durée de la polymérisation est comprise entre environ 1 h et 8h. De préférence également, toutes les étapes du procédé selon l'invention sont effectuées sous conditions stériles.

Quant à la membrane de séparation biodégradable selon l'invention, elle peut être avantageusement utilisée par un chirurgien ou par un vétérinaire pour réduire la formation d'adhérences lors de chirurgies thoraciques, pelviennes, abdominales, cardiaques ou autres.

L'hydrogel formant la membrane de séparation biodégradable peut également contenir une substance thérapeutiquement active, ou bien un système à libération contrôlée de médicament ayant une forme physiologiquement acceptable, qui convient pour une administration orale, rectale, vaginale ou sous forme d'un implant chirurgical.

Enfin, la membrane de séparation peut également être modifiée en surface par l'addition covalente de chaînes de monoéthoxy poly(éthylène-glycol) de masses moléculaires variées ou encore par incubation dans une solution de poly(éthylène-glycol) afin de modifier ses caractéristiques de biocompatibilité et de dégradabilité.

Compte tenu de ce qui précède, le nouvel hydrogel selon l'invention possède des qualités biomédicales particulièrement intéressantes, à savoir sa capacité à être sous forme d'un film mince de dimensions ajustables, sa forte teneur en eau, des propriétés mécaniques qui permettent sa manipulation aisée, le fait qu'il soit translucide ce qui facilite son positionnement, et son caractère biodégradable in situ en milieu physiologique.

La présente invention sera maintenant illustrée au moyen des exemples suivants :

### Exemple 1 : Synthèse de l'hydrogel d'albumine sérique résorbable

A titre d'exemple, une albumine sérique commerciale telle que et de manière non restrictive, celle du lapin ou du boeuf, lorsqu'elle est dissoute dans l'eau distillée à une concentration variant entre 10 et 20 % (poids/volume) et qu'une base, par exemple une solution aqueuse de 5,0N de NaOH, y est ajoutée afin d'obtenir un pourcentage final de la base se situant entre 1.5 et 0.6% (p/v); cette albumine se gélifiera suite à une période d'attente à la température de la pièce pouvant aller d'environ 1 heure jusqu'à huit heures. Cette gélification de l'albumine fournit un gel qui est hydraté, malléable, translucide et qui se révèle être un hydrogel dont le contenu en eau s'élève jusqu'au 97% (se référer au Tableau 1). La texture des hydrogels obtenus par variation de la quantité de la base et de l'albumine varie de très viscoélastique à vitreux, tout en demeurant flexible et hydraté. Le pH de l'hydrogel peut être ajusté au pH physiologique ou à un autre pH par simple incubation d'une heure dans la solution physiologique désirée. Durant cette période d'incubation et d'équilibrage, la solution tampon saline sera changée deux fois. Pour obtenir un hydrogel de dimension voulue et d'épaisseur désirée, la solution d'albumine fraîchement additionnée de la quantité nécessaire de la solution de base peut être coulée entre deux plaques de verre propres, qui sont séparées par des barres d'espacement d'épaisseur désirée (0,75 à 2,0 mm) et qui assurent de plus l'étanchéité voulue au montage. Un montage du type de celui utilisé pour préparer des gels de polyacrylamide pour électrophorèse peut être utile à cette fin.

**Tableau 1**

| Conditions de synthèse de l'hydrogel à base d'albumine sérique et caractéristiques des hydrogels obtenus. | | | | | | |
|---|---|---|---|---|---|---|
| **Quantité¹ AS** | **NaOH² 5,0N** | **Ratio %AS/%Base** | **Gélification** | **Dureté³** | **Poids après lavage⁴** | **EWC**⁵ |
| **% (p/v)** | **%(p/v) (ul** | | **+ ou -** | | **g** | **%** |
| 10 | 2.4 (120) | 4.2 | - | - | n.a. | n.a. |
| 10 | 1.2 (60) | 8.4 | ± | ± | 3.04 | 96 |
| 10 | 1.0 (50) | 10.0 | + | + | 3.72 | 97 |
| 10 | 0.8 (40) | 12.5 | + | ++ | 3.43 | 97 |
| 10 | 0.6 (30) | 16.7 | + | +++ | 3.40 | 97 |
| 17.5 | 1.2 (60) | 14.6 | + | ++++ | 2.8 | 93 |
| 17.5 | 0.6 (30) | 29.2 | + | ++++ | 4.37 | 96 |
| 20 | 1.2 (60) | 16.7 | + | ++++ | n.d. | n.d. |
| 20 | 0.6 (30) | 33.3 | + | ++++ | n.d. | n.d. |

| | | | | | | |
|---|---|---|---|---|---|---|
| ¹ solution initiale d'albumine sérique bovine (100 mg/ml d'eau (10%p/v) etc., 1 ml a été utilisé pour la synthèse des hydrogels. ² % (p/v) final de NaOH dans la solution d'albumine, le volume en micro litre de la solution aqueuse 5N de NaOH ajouté à 1 ml de la solution d'albumine est donnée entre parenthèse. ³ dureté de l'hydrogel évaluée manuellement; ± gel visqueux et difficilement manipulable, + gel très viscoélastique, ++ légèrement vitreux, +++ vitreux et ++++ très vitreux, facilement manipulable, friable sous la pression des doigts. ⁴ poids de l'hydrogel après rinçage de 1 heure dans l'eau distillé contenant 0.02% azoture de sodium comme préservatif. ⁵ EWC, % eau de l'hydrogel à équilibre= ((poids humide - poids sec théorique)/ poids humide) X 100. Le poids sec théorique correspond aux quantités d'albumine et de NaOH utilisées pour la synthèse de l'hydrogel. | | | | | | |

### Exemple 2 : Hydrogel autologue humain ou animal.

Suivant le protocole précédemment décrit, l'albumine animale est remplacée par de l'albumine humaine isolée du sang du patient ou de l'animal qui est destinée à recevoir l'hydrogel chirurgicalement. Le volume de sang à prélever sera fonction de la dimension et du nombre d'hydrogels à synthétiser. Pour ce faire, l'albumine est isolée à partir d'un volume de sang fraîchement prélevé selon la méthode de Hao (Hao, Vox. Sang. 36:313-320, 1979), donnée ici à titre d'exemple seulement. Toutes les étapes sont effectuées à une température se situant entre -5 et 0°C. En bref, cette méthode consiste en une dilution d'un volume de plasma sanguin par deux volumes d'une solution aqueuse de 0,15 M NaCl, et le pH est ajusté à 5,6 par l'addition d'une quantité d'une solution d'acétate de sodium 0,8M à pH 4.0. Une fois le mélange refroidi, une solution aqueuse d'éthanol à 95% v/v est ajoutée lentement sous agitation jusqu'à l'obtention d'une concentration finale d'éthanol de 42% (v/v). Puis l'agitation est maintenue durant une heure et la solution est centrifugée à 12,000g/1h. Le pH du surnageant est alors ajusté à 4.8 avec la solution de tampon acétate. La solution est ensuite agitée durant 1 h et est laissée sans agitation pour 3 h. La solution est par la suite centrifugée à 1 2,000g/1h. Le précipité est alors récupéré et contient l'albumine. L'albumine est dissoute dans un volume d'eau, et la quantité de protéine est évaluée par l'essai à l'acide bicinchoninique (BCA, Pierce, USA). L'albumine est diluée avec de l'eau stérile à la concentration finale désirée (entre 15 et 20% p/v), puis filtrée sur une membrane de porosité de 0.01 micron afin de la stériliser.

L'hydrogel est obtenu tel que décrit précédemment. De plus, toutes les manipulations sont effectuées de manières à ce que l'hydrogel obtenu soit stérile.

### Exemple 3 : Implantation d'un hydrogel en position péricarde-épicarde chez le lapin pour la prévention des adhérences post-opératoires

Afin d'évaluer la protection conférée par l'hydrogel contre les adhérences résultant d'une chirurgie cardiaque et pour évaluer la vitesse de résorption *in vivo* de l'hydrogel, ces derniers seront implantés dans la cavité cardiaque chez le lapin.

Pour ce faire des hydrogels d'une dimension de 25 X 20 X 1 mm sont préparés en mélangeant 150 mg d'albumine de lapin (Sigma, USA) par ml de solution 0.9% NaCl à laquelle est ajoutée 50 ml de NaOH 5.0N et une goutte de bleu de méthylène 1 % pour colorer l'hydrogel. Un fil Ethicon 910 (J&J, USA) a été ajouté préalablement entre les plaques de verres pour permettre au chirurgien de bien fixer l'hydrogel sur l'intérieur du péricarde. Après la synthèse, les hydrogels sont lavés plusieurs fois et conservés dans du NaCl 0.9%. Toutes les solutions sont filtrées sur filtre Millipore 0.2 microns et toutes les manipulations sont effectuées en conditions stériles.

Les lapins sont anesthésiés par l'isoflurane (1,5%) après prémédication avec 0,5 mg d'atropine, 0,5 mg/kg de midazolam et 6 mg/kg d'azaperone. Après relaxation musculaire avec 0.2 mg/kg de pancuronium via la voie jugulaire droite, les animaux sont intubes et ventilés mécaniquement. Une dose de 10 mg/kg de fentanyl est administrée en début de chirurgie, puis répétée avec 0,05 mg toutes les 30 minutes. Le lapin est placé en décubitus dorsal. Après rasage et désinfection du thorax une sternotomie médiane et une dissection des divers plans sont faîtes pour atterrir sur le péricarde qui est ouvert. Immédiatement, le péricarde est refermé avec une suture continue de Vicryl/0 (lapin contrôlé) ou bien, il y a mise en place de l'hydrogel d'albumine de lapin, fixation par points sur la paroi interne du péricarde et fermeture. Une hémostase et la fermeture du sternum, du tissu sous-cutané et de la peau est faite. La cicatrice est désinfectée et l'animal est réveillé.

Les animaux sont sacrifiés à 1-2-3-4-5-7-8 semaines, et la section hydrogel/péricarde prélevée pour analyse histologique. L'évaluation histologique consiste en des coupes du tissu d'intérêt préalablement fixé dans une solution de formaldéhyde neutre à 10%, suivie d'une coloration à hématoxyline-éosine. Suivant l'évaluation microscopique, le degré de fibrose et d'inflammation sera classifié selon une échelle de 0 pour absence de fibrose et de 4 lorsqu'il y a présence de cellules granulomateuses, de prolifération cellulaire etc.

Cette série d'expériences a montré qu'après 8 semaines, l'hydrogel est entièrement résorbé, qu'aucune activité inflammatoire n'est retrouvée sur le site d'implantation et que seuls des résidus d'adhésions non significatifs sont observés ça et là.

Les résultats préliminaires de l'étude in vivo, pratiquée chez le lapin, confirment donc les caractères de biocompatibilité, biodégradabilité et limitant le degré de fibrose post-opératoire. Effectivement, il n'y a pas eu de réaction de rejet à l'implantation du gel (biocompatibilité), le matériel s'est dissout en l'espace de 3 à 4 semaines (biodégradabilité) et enfin, le phénomène de protection, limitant, voire éliminant la fibrose post-opératoire, caractère le plus important, a été respecté jusqu'ici.

### Exemple 4 : Implantation sous-cutanée d'un hydrogel chez le lapin pour la libération contrôlée de médicaments et la prévention des adhérences post-opératoires

Des hydrogels de même composition de précédemment ont été synthétisés avec une dimension finale de 12.5 X 10 X 1.5 mm. Après avoir été lavés en milieu physiologique 0.9% NaCl, les hydrogels sont incubés pour 3 heures soit dans une solution d'ibuprofène 0.5% dans le NaCl 0.9%, dans une solution de 2% de poly(éthylène glycol) de masse moléculaire de 4,000 Da ou simplement dans une solution de 0.9% NaCl. Par la suite, les différents hydrogels sont implantés en sous-cutané en position dorsale chez le lapin.

Après 1-2-3-4-5-7-8 semaines, les lapins ont été sacrifiés et des coupes histologiques ont été effectuées au niveau des sites d'implantation pour évaluer les adhérences, l'inflammation et la présence de résidus d'hydrogel. Les observations suivantes ont été faites, à savoir que les résultats préliminaires confirment les caractères de bio compatibilité, biodégradabilité et un moindre degré de fibrose post-opératoire en présence des principes actifs dont les hydrogels ont étés imbibés.

### Exemple 5 : Vitesse de dissolution de l'hydrogel in vitro

Des hydrogels de différentes compositions en terme de quantité d'albumine et de base ont été préparés et lavés dans un tampon physiologique. Ils ont été incubés séparément dans une solution de tampon physiologique à 37°C pour évaluer le temps requis pour les dissolutions complètes à savoir pour déterminer le temps nécessaire à leur résorption par dissolution.

A 8 semaines, il a été observé que les gels se sont délités, ont perdu leur consistance initiale et leur résistance mécanique; de ce fait, il est constaté que la biodégradation est effective in vitro après 8 semaines, ce qui confirme l'intérêt de la présente invention.

## Revendications

1. Hydrogel physiquement réticulé, hydrophile, gonflable dans l'eau et lentement dissociable en milieu aqueux, qui consiste en des protéines sanguines d'albumine associées sous forme de gel en milieu basique.

2. Hydrogel selon la revendication 1, **caractérisé par le fait que** les protéines sanguines d'albumine sont isolées de plasma ou de sérum d'origine animale ou humaine.

3. Hydrogel selon la revendication 2, **caractérisé par le fait que** l'albumine d'origine humaine provient d'une banque du sang, est de nature autologue ou est obtenue par génie génétique.

4. Membrane de séparation biorésorbable, **caractérisée par le fait qu'**elle est formée d'un film de l'hydrogel selon l'une des revendications 1 à 3.

5. Membrane de séparation selon la revendication 4, **caractérisée par le fait que** l'hydrogel constituant ladite membrane contient une substance thérapeutiquement active.

6. Membrane de séparation selon la revendication 4, **caractérisée par le fait qu'**elle est modifée en surface, par l'addition des chaînes de monométhoxy-poly(éthylène-glycol) fixées par liaisons covalentes.

7. Membrane de séparation selon la revendication 4, **caractérisée par le fait que** l'hydrogel formant ladite membrane contient un système à libération contrôlée de médicament ayant une forme physiologiquement acceptable.

8. Procédé de préparation d'un hydrogel selon la revendication 1, qui consiste à mélanger une solution aqueuse d'albumine protéique avec une base, puis à laisser reposer le mélange jusqu'à la polymérisation des monomolécules d'albumine.

9. Procédé selon la revendication 8, **caractérisé par le fait que** la solution aqueuse d'albumine est comprise entre 10 et 20% (poids/vol.), que la base est du NaOH 5N et que le rapport entre le NaOH et la solution d'albumine est de 0.5 à 2.0% (poids/vol.), et **par le fait que** la durée de la polymérisation est comprise entre environ 1 h et 8h.

## Claims

1. A physically cross-linked, hydrophilic hydrogel, which swells in water and slowly dissociates in aqueous media, and which consists of albumin blood proteins associated in the form of a gel in a basic medium.

2. The hydrogel according to claim 1, **characterised by** the fact that the albumin blood proteins are isolated from plasma or serum of animal or human origin.

3. The hydrogel according to claim 2, **characterised by** the fact that the albumin of human origin comes from a blood bank, is autologous in nature or is obtained by genetic engineering.

4. A bioresorbable separation membrane, **characterised by** the fact that it is made up of a film of the hydrogel according to one of claims 1 to 3.

5. The separation membrane according to claim 4, **characterised by** the fact that the hydrogel that makes up said membrane contains a therapeutically active substance.

6. The separation membrane according to claim 4, **characterised by** the fact that its surface is modified by the addition of monomethoxy-poly(ethylene glycol) chains bound by covalent bonds.

7. The separation membrane according to claim 4, **characterised by** the fact that the hydrogel that makes up said membrane contains a controlled drug release system having a physiologically acceptable form.

8. A method of preparing the hydrogel according to claim 1, which consists in mixing an aqueous solution of albumin protein with a base, then allowing the mixture to rest until the albumin monomolecules are polymerized.

9. The method according to claim 8, **characterised by** the fact that the aqueous solution of albumin is between 10% and 20% (weight/volume), that the base is 5 N NaOH and that the ratio between the NaOH and the solution of albumin is 0.5% to 2.0% (weight/volume), and by the fact that the duration of polymerization is between approximately 1 hour and 8 hours.

## Patentansprüche

1. Physikalisch retikuliertes, hydrophiles, in Wasser quellfähiges und in wässrigem Milieu langsam dissoziierbares Hydrogel, das aus in Gelform in basischem Milieu verbundenen Albumin-Blutproteinen besteht.

2. Hydrogel nach Anspruch 1, **dadurch gekennzeichnet, dass** die Albumin-Blutproteine, aus Plasma oder Serum tierischen oder menschlichen Ursprungs isoliert sind.

3. Hydrogel nach Anspruch 2, **dadurch gekennzeichnet, dass** das humane Albumin aus einer Blutbank kommt, autolog ist oder durch Gentechnik gewonnen wird.

4. Biologisch resorbierbare Separationsmembran, **dadurch gekennzeichnet, dass** sie von einer Folie aus dem Hydrogel nach einem der Ansprüche 1 bis 3 gebildet wird.

5. Separationsmembran nach Anspruch 4, **dadurch gekennzeichnet, dass** das die Membran bildende Hydrogel eine therapeutisch aktive Substanz enthält.

6. Separationsmembran nach Anspruch 4, **dadurch gekennzeichnet, dass** sie auf der Oberfläche durch Hinzufügen von durch kovalente Verbindungen fixierte Monomethoxy-Poly(ethylen-glycol)-Ketten verändert ist.

7. Separationsmembran nach Anspruch 4, **dadurch gekennzeichnet, dass** das die Membran bildende Hydrogel ein System zur kontrollierten Arzneimittelfreisetzung in einer physiologisch akzeptablen Form enthält.

8. Verfahren zur Zubereitung eines Hydrogels nach Anspruch 1, das darin besteht, eine wässrige Albuminproteinlösung mit einer Base zu mischen, dann das Gemisch bis zur Polymerisation der Albumin-Monomoleküle ruhen zu lassen.

9. Verfahren nach Anspruch 8, **dadurch gekennzeichnet, dass** die wässrige Albuminlösung zwischen 10 und 20 % (Gewicht/Vol.) inklusive ist, dass die Base NaOH 5N ist und dass das Verhältnis zwischen dem NaOH und der Albuminlösung von 0,5 bis 2 % (Gewicht/Vol.) ist, und **dadurch**, dass die Dauer der Polymerisation zwischen zirka 1 Stunde und 8 Stunden inklusive ist.
